(19) Europäisches Patentamt
European Patent Office
Office européen des brevets

(11) Veröffentlichungsnummer: **0 212 115**
**B1**

(12) # EUROPÄISCHE PATENTSCHRIFT

(45) Veröffentlichungstag der Patentschrift: **21.09.88**

(51) Int. Cl.⁴: **C 07 C 121/78**, C 07 D 213/74, C 07 D 213/84, C 08 K 5/20

(21) Anmeldenummer: **86108021.6**

(22) Anmeldetag: **12.06.86**

(54) **3-t-Butyl-4-hydroxiphenylpropionsäureaminoalkylamidderivate und damit stabilisiertes organisches Material.**

(30) Priorität: **15.06.85 DE 3521558**

(43) Veröffentlichungstag der Anmeldung: **04.03.87 Patentblatt 87/10**

(45) Bekanntmachung des Hinweises auf die Patenterteilung: **21.09.88 Patentblatt 88/38**

(84) Benannte Vertragsstaaten: **CH DE FR GB IT LI**

(56) Entgegenhaltungen: **FR-A-2 358 438** **GB-A-919 711**

(73) Patentinhaber: **BASF Aktiengesellschaft, Carl-Bosch- Strasse 38, D-6700 Ludwigshafen (DE)**

(72) Erfinder: **Helwig, Reinhard, Dr., Brüsseler Ring 4, D-6700 Ludwigshafen (DE)**
Erfinder: **Neumann, Peter, Dr., Franz- Schubert-Strasse 1, D-6908 Wiesloch (DE)**
Erfinder: **Bender, Herbert, Dr., Koenigsbergstrasse 5, D-6737 Boehl- Iggelheim (DE)**
Erfinder: **Aumüller, Alexander, Dr., Brechlochstrasse 9, D-6700 Ludwigshafen (DE)**
Erfinder: **Trauth, Hubert, Milanstrasse 6, D-6724 Dudenhofen (DE)**

EP 0 212 115 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Phenolische Verbindungen, die in Nachbarstellung zur Hvdroxigruppe Alkylsubstituenten tragen, sind seit langem als Antioxidantien bekannt. Als Zusatz zu organischen Substanzen, insbesondere zu organischen Polymeren, erhöhen sie deren Stabilität gegenüber Sauerstoff beträchtlich. Gute Antioxidantien müssen einer Reihe von Anforderungen gerecht werden, wie hohe Wirksamkeit, hohe thermische Stabilität, geringe Verfärbungsneigung auch bei erhöhter Temperatur, geringe Flüchtigkeit und gute Verträglichkeit mit dem zu stabilisierenden Material. Aufgabe der Erfindung war es, Phenolderivate zu entwickeln, welche die von der Anwendung gestellten Forderungen und Kriterien erfüllen.

Gegenstand der Erfindung sind Phenolderivate der Formel (I)

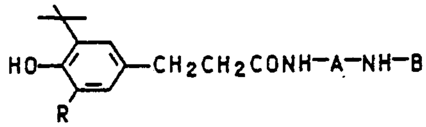

(I),

worin

R Wasserstoff, geradkettiges oder verzweigtes $C_1$- bis $C_8$-Alkyl oder worin Gycloalkyl,
A ein Brückenglied und
B ein Rest der Formel

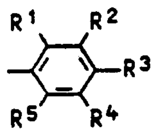 oder 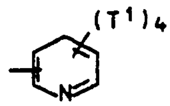 sind,

wobei $R^1$ bis $R^5$ und $T^1$ unabhängig voneinander Wasserstoff, $C_1$- bis $C_8$-Alkyl, Halogen, -CH, -$COOT^2$ oder -$CONHT^2$ bedeuten, mit der Maßgabe, daß mindestens einer der Reste $R^1$ bis $R^5$ oder $T^1$, Halogen, CN, $COOT^2$ oder $CONHT^2$ ist, und $T^2$ für Wasserstoff oder für $C_1$- bis $C_8$-Alkyl steht.

Einzelne Reste R sind z. B.

$CH_3$, $C_2H_5$, n-$C_3H_7$, i-$C_3H_7$, i-$C_4H_9$, t-$C_4H_9$, t-Amyl, i-$C_6H_{13}$, i-$C_8H_{17}$,

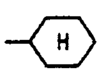 oder 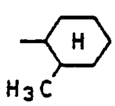, wobei, wobei Methyl, i- und t-Reste und insbesondere Methyl und t-$C_4H_9$ bevorzugt sind.

Als Brückenglieder A kommen geradkettiges oder verzweigtes $C_2$- bis $C_{16}$-Alkylen, Cycloalkylen und Cycloalkylen enthaltendes Alkylen in Betracht. Brückenglieder A sind beispielsweise -$(CH_2)_2$- , -$(CH_2)_3$-, -$(CH_2)_6$-,

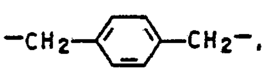, 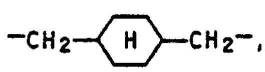, 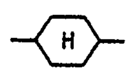 oder 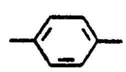, von denen

-$CH_2CH_2$- und -$CH_2CH_2CH_2$ bevorzugt sind.

Für $T^2$ sind neben Wasserstoff, z. B. $CH_3$, $C_2H_5$, $C_3H_7$, $C_4H_9$, $C_6H_{13}$ oder $C_8H_{17}$, Chlor und -CN, zu nennen.

Bevorzugte Reste $R^1$ bis $R^5$ und $T^1$ sind Wasserstoff, $C_1$- bis $C_4$-Alkyl, Chlor oder CN.

Die Synthese der erfindungsgemäßen Verbindungen kann nach bekannten Verfahren erfolgen. Am günstigsten ist es, eine Verbindung der Formel

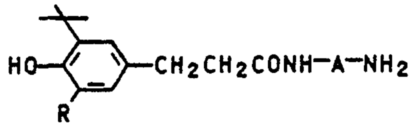

(II)

mit einer halogenhaltigen Verbindung der Formel

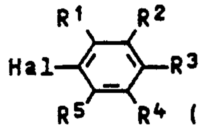 (III) oder 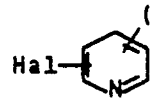 (IV)

in Gegenwart eines säurebindenden Mittels unter Abspaltung von Halogenwasserstoff umzusetzen. Als abspaltbares Halogen sind Fluor und Chlor bevorzugt.

Es ist aber auch möglich, einen Ester der Formel

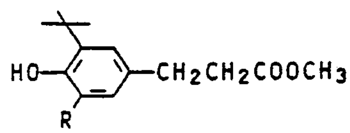

(V)

oder den entsprechenden Ethylester mit einer Aminoverbindung der Formel

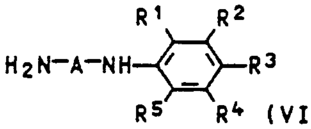

(VI)

oder

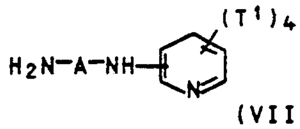

(VII)

unter Abspaltung von Methanol bzw. Ethanol umzusetzen.

Die für die entsprechenden Umsetzungen erforderlichen halogenierten Aromaten bzw. Pyridinderivate sind in der Literatur beschrieben. Die 3,5-Di-alkyl-4-hydroxiphenylpropionsäure-aminoalkylamide (II) sind in der Literatur beschrieben (z. B. DE-OS-3 500 058) oder analog herstellbar.

Einzelheiten der Herstellung können den Beispielen entnommen werden, in denen sich Angaben über Teile und Prozente, sofern nicht anders vermerkt, auf das Gewicht beziehen.

Die erfindungsgemäßen Verbindungen eignen sich zum Stabilisieren von organischem Material, insbesondere von Kunststoffen. Sie sind bei der Verwendung in Kunststoffen sowohl als Verarbeitungsstabilisatoren als auch als Langzeitstabilisatoren geeignet, Die Mittel (I) werden den zu stabilisierenden Kunststoffen in einer Konzentration von 0,01 bis 5 Gew.-%, vorzugsweise von 0,02 bis 2 Gew.-% vor, während oder nach der Polymerbildung zugesetzt.

Zum Vermischen der erfindungsgemäßen Verbindungen mit den zu stabilisierenden Kunststoffen können alle bekannten Vorrichtungen und Methoden zum Einmischen von Stabilisierungsmitteln oder anderen Zusätzen in Polymere angewandt werden.

Die durch die erfindungsgemäßen Verbindungen stabilisierten Kunststoffe können gegebenenfalls noch weitere Additive enthalten, beispielsweise Costabilisatoren, Lichtstabilisierungsmittel, Metalldesaktivatoren, antistatische Mittel, flammhemmende Mittel, Schmiermittel, Weichmacher, Vernetzungsmittel, Farbstoffe, Pigmente und Füllstoffe.

Als Costabilisatoren seien erwähnt:

Schwefel enthaltende Antioxidantien wie zum Beispiel Dilaurylthiodipropionat, Dimyristylthiodipropionat, Distearylthiodipropionat, Pentaerythrittetrakis-(β-laurylthiopropionat), Pentaerythrittetrakis-(-β-hexylthiopropionat) etc.;

Phosphor enthaltende Verbindungen wie zum Beispiel Tris-(nonyl-phenyl)- phosphit, Distearylpentaerythritdiphosphit, Tris-(2,4-di-tert.-butyl-phenyl)-phosphit, Tris-(-2-tert.-butyl-4-methylphenyl)-phosphit, Bis-(2,4-di-tert.-butylphenyl)-pentaerythritdiphosphit, Tetrakis-(2,4-di-tert.-butylphenyl)-4,4'-biphenylendiphosphit etc.

Lichtstabilisatoren, die zusammen mit den erfindungsgemäßen Verbindungen verwendet werden können, sind z. B. 2-(2'-Hydroxyphenyl)-benztriazole, 2-Hydroxybenzophenone, Arylester von Hydroxybenzoesäuren, α-Cyanozimtsäurederivate, Nickelverbindungen oder Oxalsäuredianilide, ferner Verbindungen, die eine 2,2,6,6-Tetraalkylpiperidinylgruppe enthalten, wie z. B. Sebacinsäure-bis-2,2,6,6-tetramethyl-4-piperidinylester, Sebacinsäure-bis-1,2,2, 6,6-pentamethyl-4-piperidinylester, Poly-(N,β-hydroxiethyl-2,2,6,6-tetrametyl-hydroxipiperidin-bernsteinsäureester) oder Poly- [6,[(1,1,3,3-tetramethylbutyl)-imino]-1,3,5-triazin-2,4-diyl][2-(2,2,6,6-tetramethyl-piperidinyl)-imino]-hexamethylen-[4-(2,2,6,6-tetramethylpiperidinyl)-imino].

Als organische Polymere, die durch die erfindungsgemäßen Verbindungen stabilisiert werden können, kommen z. B. in Betracht:

Polymere von Mono- und Diolefinen, wie Polyethylen niedriger oder hoher Dichte, lineares Polyethylen niedriger Dichte, Polypropylen, Polyisobutylen, Polybuten-1, Polymethylpenten-1, Polyisopren, Polybutadien sowie Copolymerisate von Mono- und Diolefinen oder Mischungen der genannten Polymeren;

Copolymerisate von Mono- oder Diolefinen mit anderen Vinylmonomeren wie z. B. Ethylen-Alkylacrylat-Copolymere, Ethylen-Alkylmethacrylat-Copolymere, Ethylen-Vinylacetat-Copolymere oder Ethyl-Acrylsäure-Copolymere; Polystyrol; Copolymere von Styrol oder α-Methylstyrol mit Dienen oder Acrylderivaten, wie Copolymere aus Styrol-Butadien, Styrol-Acrylnitril, Styrol-Ethylmethacrylat, Styrol-Butadien-Ethylacrylat und Styrol-Acrylnitril-Methylacrylat;

Halogenhaltige Polymere, wie Polyvinylchlorid, Polyvinylfluorid, Polyvinylidenfluorid sowie Copolymere mit den entsprechenden Monomeren;

Polymere die sich von α, β-ungesättigten Säuren und deren Derivaten ableiten, wie Polyacrylate und Polymethacrylate, Polyacrylamide und Polyacrylnitrile;

Polymere, die sich von ungesättigten Alkoholen und Aminen bzw. deren Acylderivaten oder Acetalen ableiten, wie Polyvinylalkohol oder Polyvinylacetat;

Polyurethane, Polyamide, Polyharnstoffe, Polyester, Polycarbonate und Polyethersulfone.

## A. Beispiele für Ausgangsverbindungen

1. 3-t-Butyl-4-hydroxi-5-methylphenylpropionsäure-2-aminoethylamid

125 g 3-t-Butyl-4-hydroxi-5-methylphenylpropionsäuremethylester und 300 g Ethylendiamin werden zusammen 5 Stunden auf 110°C erhitzt. Dann wird Vakuum angelegt und das entstandene Methanol und das überschüssige Ethylendiamin abdestilliert. Den Rückstand versetzt man mit 0,3 l Toluol und läßt eine Stunde bei Raumtemperatur stehen. Der Niederschlag wird abgesaugt und getrocknet. Er wiegt 118 g. Nach dem Umkristallisieren aus Essigester beträgt der Smp. 156 - 159 °C.

2. 3,5-Di-t-butyl-4-hydroxiphenylpropionsäure-3-aminopropylamid

146 g 3,5-Di-t-butyl-4-hydroxiphenylpropionsäuremethylester und 370 g 1,3-Propandiamin werden zusammen 11 Stunden auf 130°C erhitzt. Dann wird im Vakuum eingeengt. Der Rückstand schmilzt bei 75 - 80 °C.

3. 4-(2-Aminoethyl)amino-2,3,5,6tetrachlorbenzonitril

55 g Pentachlorbenzonitril werden bei Raumtemperatur so in 300 g Ethylendiamin eingetragen, daß die Temperatur nicht über 30°C ansteigt. Dann wird noch eine halbe Stunde bei Raumtemperatur nachgerührt. Anschließend werden langsam 0,75 l Eiswasser zugegeben, und es wird 15 Minuten nachgerührt. Der Niederschlag wird abgesaugt, mit Wasser gewaschen und getrocknet. Er wiegt 55 g. Nach dem Umkristallisieren aus Toluol unter Zusatz von Aktivkohle ist der Schmelzpunkt 140 - 144°C.

## B. Herstellbeispiele

**Beispiel 1**

4-[2-(3,5-Di-t-butyl-4-hydroxiphenylpropionamido)ethyl]amino-2,3,5,6-tetrachlorbenzonitril

27,6 g Pentachlorbenzonitril und 32 g 3,5-Di-t-butyl-4-hydroxiphenylpropionsäure-2-aminoethylamid werden mit 11 g Triethylamin in 200 g N-Methylpyrrolidon 7 Stunden bei 100°C umgesetzt. Dann werden 500 g Wasser und 400 g Toluol zugesetzt und die noch heiße Toluolphase abgetrennt und im Vakuum eingeengt. Der Rückstand wird mit 0,15 l Ethanol aufgekocht und über Nacht bei Raumtemperatur stehengelassen. Dann wird der Niederschlag abgesaugt und getrocknet. Nach dem Umkristallisieren aus Toluol schmilzt das Produkt bei 173 - 175 °C.

**Beispiel 2**

4-[-2-(3,5-Di-t-butyl-4-hydroxiphenylpropionamido)ethyl]amino-2,5,6trichlo risophthalodinitril

26,6 g Tetrachlorisophthalodinitril und 32 g 3,5-Di-t-butyl-4-hydroxiphenylpropionsäure-2-aminoethylamid werden mit 11 g Triethylamin in 200 g N-Methylpyrrolidon 2 Stunden bei 80°C umgesetzt. Nach dem Abkühlen wird 1 l Wasser zugesetzt, mit 0,5 l Toluol extrahiert, die Toluolphase getrocknet und eingeengt. Der Rückstand wird mit 0,25 l Ethanol aufgekocht und drei Stunden bei Raumtemperatur nachgerührt. Nach dem Absaugen und Trocknen des Niederschlags werden 17 g Produkt vom Smp. 209 - 211°C erhalten.

**Beispiel 3**

3-[-2-(3,5-Ditbutyl-4-hydroxiphenylpropionamido)ethyl]amino-4,5,6-trichlor-phthalsäuredinitril

26,6 g Tetrachlorphthalsäuredinitril und 32 g 3,5-Di-t-butyl-4-hydroxiphenylpropionsäure-2-aminoethylamid werden mit 11 g Triethylamin in 200 g N-Methylpyrrolidon 4 Stunden auf 80°C erhitzt. Dann werden 1 l Wasser und 0,2 l Toluol zugegeben und bei 90°C die Toluolphase heiß abgetrennt. Beim Abkühlen kristallisiert das Produkt aus. Nach Umkristallisieren aus Toluol in Gegenwart von Aktivkohle ist der Schmelzpunkt 166 - 168 °C.

**Beispiel 4**

[2-(3,5-Di-t-butyl-4-hydroxiphenylpropionamido)ethyl]amino-trichlor-nicotin-säurenitril

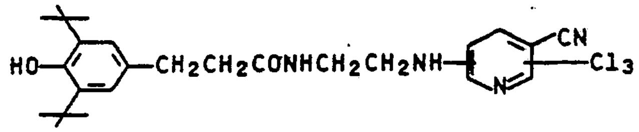

24,2 g Tetrachlornicotinsäurenitril und 32 g 3,5-Di-t-butyl-4-hydroxiphenylpropionsäure-2-aminoethylamid werden mit 11 g Triethylamin in der in Beispiel 3 beschriebenen Weise umgesetzt und aufgearbeitet. Der

Schmelzpunkt ist 188 - 193° C.

**Beispiel 5**

[2-(3,5-Di-t-butyl-4-hydroxiphenylpropionamido)-ethyl]amino-trichlorpicolinsäurenitril

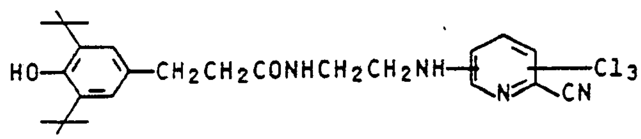

24,2 g Tetrachlorpicolinsäurenitril und 32 g 3,5-Di-t-butyl-4-hydroxiphenylpropionsäure-2-aminoethylamid werden mit 11 g Triethylamin in 0,2 l N-Methylpyrrolidon 4 Stunden bei 80° C umgesetzt. Nach Zugabe von 0,5 l Wasser und 0,4 l Toluol wird kurz bei 90° C gerührt, die heiße Toluolphase abgetrennt und eingeengt, Der Rückstand wird mit einer 1 : 1-Mischung aus Toluol und Petrolether aufgekocht und einige Stunden bei Raumtemperatur stehengelassen. Der Niederschlag wird abgesaugt und getrocknet. Er schmilzt bei 185 - 189° C.

**Beispiel 6**

[2-(3,5-Di-t-butyl-4-hydroxiphenylpropionamido)ethyl]amino-trichlorisonic otinsäurenitril

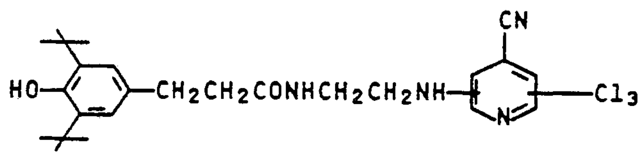

24,2 g Tetrachlorisonicotinsäurenitril und 32 g 3,5-Di-t-butyl-4-hydroxiphenylpropionsäure-2-aminoethylamid werden mit 11 g Triethylamin in 0,2 l N-Methylpyrrolidon in der in Beispiel 2 beschriebenen Weise umgesetzt. Der eingeengte Toluolextrakt wird aus einer Mischung aus Cyclohexan und Toluol im Verhältnis 4 : 1 unter Zusatz von Aktivkohle umkristallisiert. Der Schmelzpunkt ist 150 - 160° C.

**Beispiel 7**

3,5-Di-t-butyl-4-hydroxiphenylpropionsäure-2-(tetrachlorpyridinamino) ethylamid

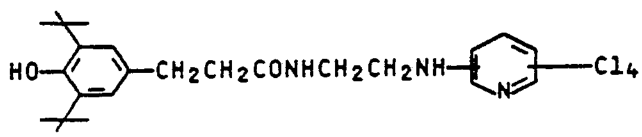

25,2 g Pentachlorpyridin und 32 g 3,5-Di-t-butyl-4-hydroxiphenylpropion-säure-2-aminoethylamid werden mit 11 g Triethylamin in 0,2 l N-Methylpyrrolidon 4 Stunden bei 50° C umgesetzt. Nach Zugabe von 1 l Wasser wird mit 0,3 l Toluol extrahiert, der Extrakt über Aktivkohle filtriert und im Vakuum eingeengt. Der Rückstand wird in einer Ethanol/Wasser-Mischung gelöst und bis zur Kristallisation stehengelassen Der getrocknete Niederschlag schmilzt bei 117 - 122° C.

**Beispiel 8**

6-[2-(3,5-Di-t-bytyl-4-hydroxiphenylpropionamido)ethyl]amino-2-chlor-4 -methyl-nicotinsäurenitril

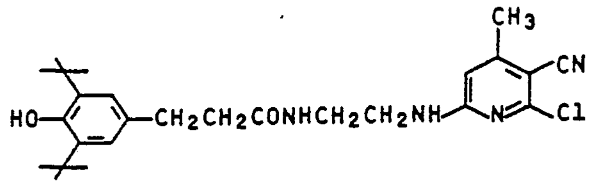

18,7 g 2-6-Dichlor-4-methylnicotinsäurenitril und 32 g 3,5-Di-t-butyl-4-hydroxiphenylpropionsäure-2-aminoethylamid werden mit 10,1 g Triethylamin in 0,2 l N-Methylpyrrolidon 8 Stunden bei 50°C umgesetzt. Dann werden 1 l Wasser und 0,4 l Toluol zugesetzt, die Toluolphase abgetrennt, getrocknet, mit Aktivkohle filtriert und eingeengt. Der Rückstand wird in 100 ml Ethanol heiß gelöst und die Lösung langsam mit 1 l Petrolether versetzt. Nach dem Abkühlen wird der Niederschlag abgesaugt und getrocknet. Er schmilzt bei 162 - 165°C.

**Beispiel 9**

4-[2-(3,5-Di-t-butyl-4-hydroxiphenylpropionamido)ethyl]amino-benzo-nitril

13,75 g 4-Chlorbenzonitril und 32 g 3,5-Ditbutyl-4-hydroxiphenylpropionsäure -2-aminoethylamid werden mit 12,9 g Chinolin in 0,2 l N-Methylpyrrolidon 8 Stunden bei 180°C umgesetzt. Darin wird abgekühlt, in 0,5 l 7 %-ige Salzsäure eingerührt, mit 0,2 l Toluol extrahiert, der Toluolextrakt eingeengt und der Rückstand über eine Kieselgel-Säule (Laufmittel: Essigester) gereinigt. Das Produkt schmilzt bei 146 - 148°C.

**Beispiel 10**

2-Chlor-6-[2-(3,5-di-t-butyl-4-hydroxiphenylpropionamido)ethyl]amino-benzonitril

17,2 g 2,6-Dichlorbenzonitril und 32 g 3,5-Di-t-butyl-4-hydroxiphenylpropionsäure-2-aminoethylamid werden in 150 ml Chinolin 4 Stunden bei 200°C umgesetzt. Nach dem Abkühlen wird die Lösung in 1 l 10 %-ige Salzsäure eingerührt, mit 0,4 l Toluol extrahiert und die getrocknete Toluolphase eingeengt. Reste an flüchtigen Bestandteilen werden durch 2-stündiges Erhitzen auf 180°C im Ölvakuum entfernt. Der Rückstand wird säulenchromatographisch gereinigt (Kieselgel/Methylenchlorid). Das Produkt schmilzt bei 164 - 167°C.

Analog den Angaben in den Beispielen 1 bis 10 wurden die folgenden Verbindungen (I) hergestellt;

**Tabelle 1:** Verbindungen der Formel I

| Beispiel Nr. | R | A | B |
|---|---|---|---|
| 11 | $CH_3$ | $(CH_2)_2$- | 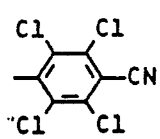 |
| 12 | $CH_3$ | $-(CH_2)_3$- | 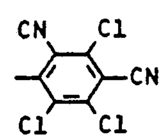 |
| 13 | $CH_3$ | $-(CH_2)_2$- | 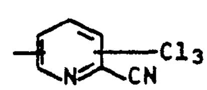 |
| 14 | t-$C_4H_9$ | $-(CH_2)_3$- | 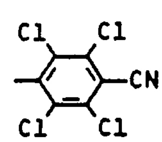 |
| 15 | t-$C_4H_9$ | $-(CH_2)_2$- | 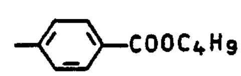 |

## C. Anwendungsbeispiele

C1. Prüfung der Langzeitstabilität von Polypropylen

0,1 % Calciumstearat enthaltendes Polypropylen (z. B. Novolen® 1100) wird mit 0,1 % der in der Tabelle 2 angegebenen Verbindungen als Antioxidans in einem Schnellmischer homogenisiert und über einen Laborextruder Typ ET 20 der Firma Weber bei einer Massetemperatur von 220°C extrudiert und granuliert. Aus dem Granulat werden bei 220°C Probekörper vom Format 20 x 20 x 1 mm gepreßt und bei 149°C in einem Umluftwärmeschrank mit Frischluftzufuhr gelagert.

Gemäß DIN 53383 wird als Oxidationsstabilitätszeit die Zeit bis zum Eintreten der sogenannten lokalen Versprödung ermittelt. Einige Beispiele sind in Tabelle 2 zusammengefaßt.

**Tabelle 2:**

| Verbindung aus Beispiel | Oxidationsstabilitätszeit [h] | Farbe bei beginnender Versprödung |
|---|---|---|
| 1 | 680 | blaßgelblich |
| 2 | 530 | blaßgelb |
| 5 | 520 | blaßgelb |
| 6 | 460 | bräunlich |
| 8 | 400 | gelblich |

C2. Prüfung der Verarbeitungsstabilität von Polypropylen

0,1 % Calciumstearat enthaltendes Polypropylen (z. B. Novolen 1100) wird mit 0,1 % der in Tabelle 3 angegebenen Verbindung in einem Schnellmischer homogenisiert und über einen Laborextruder Typ ET 20 der Firma Weber bei einer Massetemperatur von 250°C extrudiert und granuliert. Das Granulat wird weiteren sieben Extruderpassagen unterworfen. Nach jeder Extruderpassage wird eine Granulatprobe entnommen und deren Schmelzindex MFI 230°C/2160 g [g/10 min] nach DIN 53735 gemessen.

**Tabelle 3:**

| Verbindung aus Beispiel | Schmelzindex [g/10 min] nach Extruderpassage Nr. | | | |
|---|---|---|---|---|
| | 1 | 3 | 5 | 8 |
| 1 | 4,63 | 7,2 | 10,1 | 14,45 |
| 2 | 4,6 | | | 15,6 |
| ohne Antioxidans | 5,4 | 9,5 | 17,0 | 26,0 |

C3.Prüfung des Gas-fadings

0,1 % Calciumstearat enthaltendes Polypropylen (z. B. Novolen 1100-Typ) wird mit 0,1 % der in Beispiel 1 beschriebenen Verbindung in einem Schnellmischer homogenisiert und über einen Laborextruder Typ ET 20 der Firma Weber bei einer Massetemperatur von 250°C extrudiert und granuliert. Das Granulat wird auf einer Spinnapparatur zu Fasern (ca. 30 dtex) versponnen und jeweils eine Probe von etwa 10 g dieser Fasern 10 Minuten lang in einem Brennkasten (DIN 52906) den Abgasen einer Propangasflamme ausgesetzt. Die Flamme wird so eingestellt, daß die Temperatur in der direkten Umgebung der Fasern ca. 110°C beträgt. Nach der Gaseinwirkung wird die aufgetretenen Farbtonänderungen beurteilt.

| Stabilisator | Faserfärbung nach Gaseinwirkung |
|---|---|
| Verbindung aus Beispiel 1 | farblos |
| Irganox® 1010 | rosa |
| ohne Stabilisator | hellgrau |

**Patentansprüche**

1. Phenolderivate der allgemeinen Formel (I)

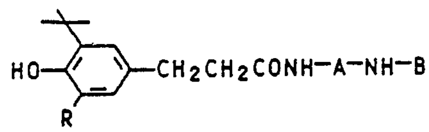

(I),

worin

R Wasserstoff, geradkettiges oder verzweigtes $C_1$- bis $C_8$-Alkyl oder Cycloalkyl,
A ein Brückenglied und
B ein Rest der Formel

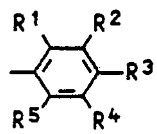

oder

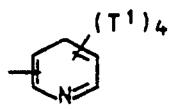

sind,

wobei $R^1$ bis $R^5$ und $T^1$ unabhängig voneinander Wasserstoff, $C_1$-bis $C_8$-Alkyl, Halogen, -CN, $COOT^2$ oder $CONHT^2$ bedeuten, mit der Maßgabe, daß mindestens einer der Reste $R^1$ bis $R^5$ oder $T^1$ Halogen, CN, $COOT^2$ oder $CONHT^2$ ist, und $T^2$ für Wasserstoff oder für $C_1$- bis $C_8$-Alkyl steht.

2. Verbindungen gemäß Anspruch 1, in denen R eine Methyl- oder t-Butylgruppe bedeutet.

3. Verbindungen gemäß Anspruch 1 oder 2, in denen A ein geradkettiges oder verzweigtes $C_2$ bis $C_{16}$-Alkylen, Cycloalkylen oder Cycloalkylen enthaltendes Alkylen ist.

4. Verbindungen gemäß Anspruch 3, in denen A für $-CH_2CH_2-$ oder $-CH_2CH_2CH_2-$ steht.

5. Verbindungen gemäß Anspruch 1, 2, 3 oder 4, in denen $R^1$ bis $R^5$ und $T^1$ unabhängig voneinander für Wasserstoff, $C_1$- bis $C_4$-Alkyl, Chlor oder CN stehen.

6. Verbindungen gemäß Anspruch 5, in denen $R^1$ bis $R^5$ und $T^1$ unabhängig voneinander für Wasserstoff, Methyl, Chlor oder -CN stehen,

7. Verwendung der Verbindungen gemäß der Ansprüche 1 bis 6 zum Stabilisieren von organischem Material.

8. Verwendung der Verbindungen gemäß den Ansprüchen 1 bis 6 zum Stabilisieren von organischen Polymeren.

9. Verwendung der Verbindungen gemäß Anspruch 8 zum Stabilisieren von Polyolefinen.

**Claims**

1. A phenol derivative of the formula (I)

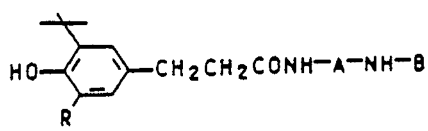

(I)

where R is hydrogen, straight-chain or branched $C_1$-$C_8$-alkyl or cycloalkyl, A is a bridge member and B is a radical of the formula

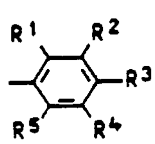

or

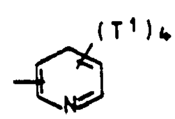

where $R^1$ to $R^5$ and $T^1$ independently of one another are each hydrogen, $C_1$-$C_8$-alkyl, halogen, CN, $COOT^2$ or $CONHT^2$, with the proviso that one or more of the radicals $R^1$ to $R^5$ and $T^1$ are halogen, CN, $COOT^2$ or $CONHT^2$, and $T^2$ is $C_1$-$C_8$-alkyl.

2. A compound as claimed in claim 1, wherein R is methyl or tert-butyl.

3. A compound as claimed in claim 1 or 2, wherein A is straight-chain or branched $C_2$-$C_{16}$-alkylene, cycloalkylene or cycloalkylene-containing alkylene.

4. A compound as claimed in claim 3, wherein A is $-CH_2CH_2-$ or $-CH_2CH_2CH_2-$.

5. A compound as claimed in claim 1, 2, 3 or 4, wherein $R^1$ to $R^5$ and $T^1$ independently of one another are each hydrogen, $C_1$-$C_4$-alkyl, chlorine or CN.

6. A compound as claimed in claim 5, wherein $R^1$ to $R^5$ and $T^1$ independently of one another are each hydrogen, methyl, chlorine or CN.

7. Use of a compound as claimed in any of claims 1 to 6 for stabilizing organic material.

8. Use of a compound as claimed in any of claims 1 to 6 for stabilizing organic polymers.

9. Use of a compound as claimed in claim 8 for stabilizing polyolefins.

**Revendications**

1. Dérivés phénoliques de formule générale (I)

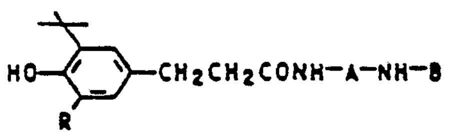

(I),

dans laquelle

R est un atome d'hydrogène, un radical alkyle en $C_1$ à $C_8$ à chaîne droite ou ramifiée ou un radical cycloalkyle,

A est un maillon de pontage et

B est un radical de formule

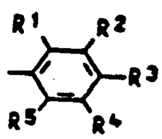

ou

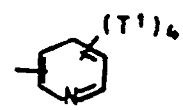

$R^1$ à $R^6$ et $T^1$ représentant, indépendamment les uns des autres, des atomes d'hydrogène, des radicaux alkyle en $C_1$ à $C_8$, des atomes d'halogène, -CN, $COOT^2$ ou $CONTH^2$, avec cette condition que l'un au moins des radicaux $R^1$ à $R^5$ ou $T^1$ soit un atome d'halogène, -CN, $COOT^2$ ou $CONTH^2$, $T^2$ étant mis pour un radical alkyle en $C_1$ à $C_8$.

2. Composés selon la revendication 1, dans lesquels R représente un groupe méthyle ou t-butyle.

3. Composés selon la revendication 1 ou 2, dans lesquels A est un radical alkylène en $C_2$ à $C_{16}$ à chaîne droite ou ramifiée, cycloalkylène ou alkylène contenant un groupe cycloalkylène.

4. Composés selon la revendication 3, dans lesquels A est mis pour $-CH_2CH_2-$ ou $-CH_2CH_2CH_2-$.

5. Composés selon l'une quelconque des revendications 1 à 4, dans lesquels $R^1$ à $R^5$ et $T^1$ sont mis, indépendamment les uns des autres, pour des atomes d'hydrogène, des radicaux alkyle en $C_1$ à $C_4$, des atomes de chlore ou pour -CN.

6. Composés selon la revendication 5, dans lesquels $R^1$ à $R^5$ et $T^1$ sont mis, indépendamment les uns des autres, pour des atomes d'hydrogène, des radicaux méthyle, des atomes de chlore ou pour -CN.

7. Utilisation des composés selon l'une quelconque des revendications 1 à 6 pour la stabilisation de matières organiques.

8. Utilisation des composés selon l'une quelconque des revendications 1 à 6 pour la stabilisation de polymères organiques.

9. Utilisation des composés selon la revendication 8 pour la stabilisation de polyoléfines.